# EUROPEAN PATENT APPLICATION

(11) **EP 0 577 034 A2**
(43) Date of publication of application: **05.01.1994**
(21) Application number: 93110239.6
(22) Date of filing: 26.06.1993
(51) Int. Cl.: A23K 1/16, A23K 1/18

(54) **Composition to be fed to fish and shellfish and method for preventing pollution of water for raising fish and shellfish**

(30) Priority: 30.06.1992 JP 173124/92
(71) Applicant: Senju Pharmaceutical Co., Ltd., Osaka-shi, Osaka 541 (JP)
(72) Inventor: Morita, Yasushi, Toyonaka-shi, Osaka 560 (JP)
(74) Representative: von Kreisler, Alek, Dipl.-Chem.

(57) **Abstract**

A composition to be fed to fish and shellfish, which comprises a polymer compound capable of liquid-gel phase transition in the water for raising fish and shellfish, by change of ionic strength or change of pH, and a method for suppressing pollution of the water for raising fish and shellfish by using the composition of the invention. The composition of the present invention permits effective administration of feed, nutrition furnisher, and agent for treatment and prevention of diseases while suppressing water pollution.

## Description

### TECHNICAL FIELD OF THE INVENTION

The present invention relates to a composition to be fed to fish and shellfish, which comprises a polymer compound capable of liquid-gel phase transition in the water for raising fish and shellfish, such as sea water or fresh water.

### BACKGROUND OF THE INVENTION

Conventionally, various substances for fish and shellfish, such as feed, nutrition furnishers, and agents for treating and/or preventing diseases of fish and shellfish have been commercially available. However, these substances have various defects such as those to be mentioned below.

The feed for fish and shellfish which is on the market includes, for example, natural feed such as benthic animals (e.g. water flea, rotifer), tubiflex, brine shrimp, and feed worms, as well as artificial feed in flakes, granules, or liquids, such as mixed feed of fish meal, shrimp meal, and grain meal (e.g. soybean). It is most ideal for the feed in such forms to be fed several times a day in an amount suitably eaten up in 2 or 3 minutes. Practically speaking, however, such feeding mode is very difficult, and the amount of feed mostly exceeds the limit fish and shellfish can take in. Once fed in an amount beyond intake capacity of fish and shellfish, the excess feed dissolves in water and floats or sinks, thus giving rise to water pollution.

Nutrition furnishers for fish and shellfish include, for example, vitamins and amino acids. Such nutrition furnishers are generally admixed with feed mixture, but are susceptible to the defects as mentioned above. In addition, the use of a nutrition furnisher alone results in its dissolution in or eluting out into water. It is thus extremely difficult to orally feed nutrition furnishers effectively by either of these methods.

When antibiotics and synthetic antibacterial agents are used for the treatment and prevention of diseases in fish and shellfish, these drugs are administered to fish and shellfish upon mixing with feed, or by way of a drug bath prepared by adding a drug into a water tank. These methods also have many defects such that water can be polluted, and administration of a precisely effective amount of a drug to fish and shellfish is extremely difficult.

### SUMMARY OF THE INVENTION

In view of the situation as described, the present inventor conducted intensive studies with the aim of providing a composition which permits effective feeding of feed, nutrition furnisher, and agent for the treatment and prevention of diseases to fish and shellfish (hereinafter sometimes referred to merely as administration substances), while suppressing pollution of the water for raising fish and shellfish. As a result, the inventor has found that addition of a polymer compound capable of liquid-gel phase transition in the water for raising fish and shellfish, to the aforementioned administration substances can overcome the defects seen when conventional administration substances per se are used, by the action of the polymer compound which underwent liquid-gel phase transition to entrap said administration substances, thereby keeping the administration substances from dissolving in and eluting out into water, and that said administration substances can be effectively fed to fish and shellfish, which resulted in the completion of the invention.

### DETAILED DESCRIPTION OF THE INVENTION

Accordingly, the present invention relates to a composition to be fed to fish and shellfish, which comprises a polymer compound capable of liquid-gel phase transition in the water for raising fish and shellfish, such as sea water or fresh water. More specifically, the present invention relates to a composition to be fed to fish and shellfish, which comprises a polymer compound capable of liquid-gel phase transition induced by change of ionic strength or change of pH, and administration substances for fish and shellfish.

Also, the present invention relates to a method for suppressing pollution of the water for raising fish and shellfish, which comprises adding a polymer compound capable of liquid-gel phase transition by change of ionic strength or change of pH, and administration substances for fish and shellfish, to the water for raising fish and shellfish.

The composition of the present invention to be fed to fish and shellfish can be suitably used for fish in sea water and fish in fresh water. Also, the composition of the present invention to be fed to fish and shellfish can be used for cultured fish and fish for appreciation, and also, it can be used as scattered bait as appropriate for attracting fish.

Examples of fish and shellfish include horse mackerel, sea bass, sea bream, butterfly fish, anemone fish, carp, pond smelt, eel, goldfish, guppy, common octopus, arrow squip, lobstar, short-neck clam, and so on.

The composition to be fed to fish and shellfish of the present invention may be prepared into solution, suspension, or solid agents such as powders and granules which may be dissolved or suspended in purified water when in use. The preparation method therefor may be any convenient means which has been conventionally employed in the fields of pharmaceuticals, agricultural chemicals, and so on.

The composition of the present invention is fed in the form of solution, suspension, or solid dissolved in purified water, etc. prepared as above, by adding same in the water for raising fish and shellfish. The water for raising fish and shellfish includes, for example, sea water (e.g. natural sea water, artificial sea water), fresh water (e.g. river water, pond water, lake water, rainwater), water to be used for fish culture, and water for keeping fish for appreciation.

The polymer compound to be added to the composition for fish and shellfish of the present invention is capable of liquid-gel phase transition in the water for raising fish and shellfish. More specifically, the compound is preferably that capable of liquid-gel phase transition with increasing ionic strength or changing pH. The polymer compound which undergoes gelation caused by an increased ionic strength is selected as appropriate according to the ionic strength of the water for raising fish and shellfish to be used. The ionic strength which causes gelation of the polymer compound is preferably not less than 0.2, and when the water for raising fish and shellfish is sea water, it is generally not less than 0.5, preferably 0.6-0.8.

The polymer compound which undergoes gelation as a result of pH change is those which transit to gel phase at a pH of the water for raising fish and shellfish of from about 5 to 9, preferably from about 6 to 8.

When undergoing liquid-gel phase transition, the polymer compound to be added to the composition for fish and shellfish of the present invention is formed into particles in which feed, nutrition furnisher, and agent for the treatment and prevention of diseases are entrapped, thereby effectively preventing those components from dissolving in or eluting out into water. The polymer compound to be contained in the composition for fish and shellfish of the present invention desirably has properties physiologically acceptable to fish and shellfish.

Examples of the polymer compound to be added to the composition for fish and shellfish of the present invention, and which is capable of liquid-gel phase transition due to change of the ionic strength of the water for raising fish and shellfish include polysaccharides such as alginic acid, its salt, κ-carageenan, and gellan gum. These become gels by the presence of sodium ion, potassium ion, calcium ion, or the like at the aforementioned ionic strength. These polymer compounds are usually used in sea water, and appropriately selected according to the ionic strength of the sea water to be used.

Examples of the polymer compound to be used for the composition for fish and shellfish of the present invention, and which is capable of liquid-gel phase transition according to pH change advantageously include acrylic resin, polyvinyl esters, and cellulose derivatives (e.g. cellulose esters, hydroxyalkylcelluloses). The acrylic resin is exemplified by a copolymer such as methacrylic acid-methacrylic acid ester copolymer, methacrylic acid-acrylic acid ester copolymer, or methacrylic acid copolymer, and examples of commercially available ones include Eudragit E, Eudragit L, and Eudragit S (trademark, manufactured by Rohm Pharma). Examples of the polyvinyl esters include polyvinyl acetal diethylaminoacetate and polyvinyl acetate phthalate; examples of the cellulose esters include cellulose acetate phthalate and cellulose acetate; and examples of hydroxyalkylcellulose include hydroxypropylmethylcellulose phthalate and hydroxypropylmethylcellulose acetate succinate. Since these polymers have carboxylic acid group, hydroxyl group, or amino group in molecules, addition of alkali or acid in an amount equivalent to or exceeding the amount of these functional groups in molecules makes it possible for them to exist in a solution state. An aqueous solution of the polymer compound thus prepared becomes gel according to the pH of fresh water or sea water. These polymer compounds are appropriately mixed for use according to the object of the invention.

While the amount of the polymer compound to be added to the composition for fish and shellfish of the present invention is suitably determined depending on the kind thereof, the kinds of feed, nutrition furnisher, and agent for the treatment and prevention of diseases, it is normally about 0.05-10 w/v%, preferably about 0.1-5 w/v%.

When the composition to be fed to fish and shellfish of the present invention is prepared into feed, other feed to be combinedly used includes, for example, one or more from fish meal, shrimp meal, grain meal such as soybean, and yeast in mixture, or extracts thereof.

When the composition to be fed to fish and shellfish of the present invention is prepared into a nutrition furnisher, the nutrients to be contained are, for example, amino acid, vitamin, and fatty acid solely or in combination. Also, glycyrrhizin may be used as an agent for strengthening liver.

Furthermore, when the composition to be added to fish and shellfish of the present invention is prepared into an agent for treating and preventing diseases, drugs such as sulfanilamides (e.g. sulfamonomethoxine), antibiotics (e.g. ampicillin, oxytetracycline, kanamycin), and synthetic antibacterial agents (e.g. nalidixic acid) may be used solely or in combination.

The composition to be fed to fish and shellfish of the present invention may be prepared by simultaneously mixing the aforementioned feed, nutrition furnisher, and agent for treating and preventing diseases.

In addition, the composition to be fed to fish and shellfish of the present invention may comprise an agent which attracts feeding of fish and shellfish in terms of flavor, color, etc. so as to make the fish and shellfish desire to capture the feed. The feeding attractant is preferably nucleic acid-related substances such as inosinic acid. As the flavor, preferred are ground roe of cod fish, ground roe of brine shrimp, squid oil, and fish feed flavor. The coloring material is preferably one which is nontoxic to fish and shellfish, with particular preference given to dyes approved as food additives, such as food dye red 102, food dye yellow 4, and food dye blue 2. Besides these, there may be used astaxanthine which is a natural colorant widely distributed in aquatic animals such as in shells of Crustacean, salmon meat, and the epidermis of alfonsin, as a coloring material for increasing economical value of salmon, sea bream, etc.

The composition to be fed to fish and shellfish of the present invention may comprise preservatives, stabilizers, emulsifiers, and pH adjusters as necessary. Examples of the preservatives include sorbic acid and p-hydroxybenzoic acid, and examples of stabilizers include disodium ethylenediaminetetraacetate, erythorbic acid, and sodium erythorbate. As the emulsifiers, those approved as food additives such as sorbitan monolaurate, glyceryl monostearate, sucrose acetate isobutylate, and lecithin may be used. Examples of the pH adjusters include sodium hydroxide and hydrochloric acid.

The present invention is hereinbelow explained in detail by illustrating experiment examples and examples so as to clarify the effect of the invention. It is to be understood that these examples are for exemplification purpose only, and do not limit the scope of the invention.

### Experiment 1

After κ-carageenan (0.4 g) was dissolved in 80 ml of purified water by heating, fish meal (1.0 g) was suspended therein, and the total amount was made 100 ml to give a κ-carageenan composition. Separately, 1 g of fish meal was suspended in 100 ml of purified water for use as a control. One ml each of the κ-carageenan composition or control was added to 4 ml of artificial sea water prepared by dissolving MARINE ART (trademark, manufactured by Senju Pharmaceutical Co., Ltd., Japan), and properties were observed. Also, the transmittance of the artificial sea water was determined at a wavelength of 600 nm after the addition of the κ-carageenan composition or control and evaluated on the basis of the transmittance of the artificial sea water without addition as 100% to determine turbidity of the artificial sea water caused by the fish meal, the results of which are summarized in Table 1.

**Table 1**

| Transmittance of the artificial sea water added with κ-carageenan composition | |
|---|---|
| Sample | Transmittance (%)¹⁾ |
| Control | 4.8 |
| κ-Carageenan composition | 97.8 |

| | |
|---|---|
| Note : 1) Transmittance of artificial sea water at a wavelength of 600 nm, which was filtered through gauze 8 hours after the addition of κ-carageenan composition or control. | |

As a result, it was found that addition of the κ-carageenan composition to artificial sea water immediately caused gelation of the composition, and the gel floated in the artificial sea water. The gel state was maintained even 8 hours later. As is evident from the results given in Table 1, transmittance of the artificial sea water added with control was poor with high turbidity of the sea water. In contrast, transmittance of the artificial sea water added with the κ-carageenan composition was high even at 8 hours after the addition, scarcely indicating turbidity caused by the fish meal.

### Experiment 2

Gellan gum (0.2 g) and food dye blue 2 (0.05 g) were mixed, added with 80 ml of purified water and heated for dissolution, and the total amount was made 100 ml to give a gellan gum composition. One ml of this solution was added to 4 ml of artificial sea water prepared by dissolving MARINE ART (trademark, manufactured by Senju Pharmaceutical Co., Ltd., Japan). Eight hours later, the artificial sea water was filtered, and the absorbance of the food dye blue 2 in the filtrate was determined at a wavelength of 610 nm. As a control, used was a mixture of 1 ml from a solution-prepared by dissolving 0.05 g of the food dye blue 2 in 100 ml of purified water, and 4 ml of artificial sea water. The results are shown in Table 2.

**Table 2**

| Leakage of colorant from gellan gum composition | |
|---|---|
| Sample | Absorbance¹⁾ |
| Control | 0.489 |
| gellan gum composition | 0.079 |

| | |
|---|---|
| Note : 1) Absorbance of artificial sea water at a wavelength of 610 nm, which was filtered 8 hours after the addition of gellan gum composition or control. | |

As a result, it was found that addition of the gellan gum composition to artificial sea water immediately caused formation of blue gel, and the gel floated in the artificial sea water. As is evident from the results given in Table 2, absorbance of the artificial sea water added with the gellan gum composition at 8 hours from the addition of the composition was low and leakage of the colorant from the gel composition of gellan gum was not observed.

### Experiment 3

Eudragit E (8 g, manufactured by Rohm Pharma) was dissolved in 1N hydrochloric acid (10 ml) and ethanol (90 ml), and the solvent was evaporated under reduced pressure. Purified water (80 ml) was added to the residue, and after dissolution, food dye red 102 (0.05 g) was added and dissolved. The total amount was made 100 ml with purified water to give an Eudragit E composition. One ml of this solution was added to 4 ml of 0.1 M phosphate buffer, pH 7, and 4 hours later, the 0.1 M phosphate buffer was filtered and the absorbance of the food dye 102 in the filtrate was determined at a wavelength of 510 nm. As a control, used was a mixture of 1 ml from a solution prepared by dissolving 0.05 g of food dye red 102 in 100 ml of purified water, and 4 ml of 0.1 M phosphate buffer, pH 7. The results are shown in Table 3.

**Table 3**

| Leakage of colorant from Eudragit E composition | |
|---|---|
| Sample | Absorbance¹⁾ |
| Control | 0.618 |
| Eudragit E composition | 0.248 |

| | |
|---|---|
| Note : 1) Absorbance at a wavelength of 510 nm upon filtration at 4 hours after the addition of the Eudragit E composition or control to 0.1 M phosphate buffer, pH 7. | |

As a result, it was found that addition of the Eudragit E composition to 0.1 M phosphate buffer, pH 7, caused formation of red gel, and the gel floated in the 0.1 M phosphate buffer, pH 7. As is evident from the results given in Table 3, the absorbance of the 0.1 M phosphate buffer, pH 7, at 4 hours after the addition of the Eudragit E composition was low and leakage of the colorant from the Eudragit E composition gel was suppressed.

### Example 1

### Feed composition for the fish in sea water

κ-Carageenan (0.4 g) was dissolved by heating in 80 ml of purified water, and thereto was added 1.0 g of fish meal, 0.02 g of sorbic acid, and 0.1 g of ground roe of brine shrimp. The total amount was made 100 ml, and the mixture was used as a feed composition for sea water fish for appreciation.

### Example 2

### Feed composition for fish

Eudragit L (4 g) was dissolved in 80 ml of ethanol, and thereto were added 10 ml of 1N sodium hydroxide, 0.5 g each of fish meal and shrimp meal, 0.02 g of sorbic acid, and 0.1 ml of fish feed flavor, followed by mixing. Ethanol was added to make the total amount 100 ml. The solvent was evaporated under reduced pressure, and the resultant mixture was dried and pulverized into powder to give a feed composition.

### Example 3

### Amino acid furnisher for sea water fish

Sodium alginate (1 g), combined amino acid [sodium aspartate (1.47 g), threonine (0.45 g), serine (0.5 g), gultamic acid (2.28 g), proline (0.8 g), glycine (0.62 g), alanine (0.69 g), valine (0.31 g), methionine (0.23 g), isoleucine (0.5 g), leucine (1.0 g), phenylalanine (0.49 g), histidine (0.26 g), lysine (0.76 g), arginine (0.64 g)], and ground roe of cod fish (1.0 g) were thoroughly mixed in advance, and the mixture was dissolved by heating in 80 ml of purified water. The total amount was made 100 ml and the feed was used for furnishing amino acid to sea water fish.

The formulation of various amino acids in the above-mentioned combined amino acid was determined according to the amino acid analysis of the Mayer's diet comprised in an artificial diet determined by Katayama et al. [Bull. Japan. Soc. Sci. Fish., *46*, 237-245 (1980)].

### Example 4

### Vitamin furnisher for cultured fish

Hydroxypropylmethylcellulose phthalate (5 g) was dissolved in 80 ml of ethanol, and thereto were added 5 ml of 1N sodium hydroxide, combined vitamin [vitamin A (1330 IU), vitamin D₃ (550 IU), α-tocopherol (10 IU), phytonadione (3 mg), thiamine hydrochloride (2.8 mg), biotin (0.8 mg), folic acid (2 mg), cyanocobalamin (0.003 mg), inositol (69.1 mg)], lecithin (1 g), erythorbic acid (15 mg), and squid oil (0.1 ml). Ethanol was added to make the total amount 100 ml. The solvent was evaporated under reduced pressure, and the resultant mixture was dried and pulverized into powder to give a feed for furnishing vitamin to cultured fish.

The formulation of various vitamins in the above-mentioned vitamin was determined according to the vitamin added in the Tunison modified trout diet, as disclosed in Katayama et al. [Bull. Japan. Soc. Sci. Fish., *46*, 237-245 (1980)].

### Example 5

### Nutrition furnisher A for cultured fish

Eudragit E (4 g) was dissolved in 13 ml of 1N hydrochloric acid, and thereto was added purified water to make the total amount 80 ml. Then, the combined amino acid as described in Example 3, thiamine (0.1 g), biotin (0.01 g), and pantothenic acid (0.1 g) were added thereto, dissolved therein, and the pH of the mixture was adjusted to 5 with 1N sodium hydroxide. Further, 0.1 g of ground roe of brine shrimp was added thereto, and the mixture was lyophilized and pulverized into powder to give a nutrition furnisher A for cultured fish.

### Example 6

### Nutrition furnisher B for cultured fish

Sodium alginate (1 g), the combined amino acid as described in Example 3, thiamine hydrochloride (0.1 g), biotin (0.01 g), and pantothenic acid (0.1 g) were thoroughly mixed, and purified water (50 ml) was added thereto, whereafter the mixture was dissolved by heating. Separately, Eudragit L (2 g) was dissolved in 1N sodium hydroxide (23 ml) and mixed with the mixture prepared in advance. Then, the pH of the mixture was adjusted to 6 with 1N hydrochloric acid. Fish feed flavor (0.1 ml) was added thereto, and the total amount was made 100 ml to give a nutrition furnisher B for cultured fish.

### Example 7

### Agent for strengthening liver of cultured fish

To Eudragit S (4 g) dissolved in 80 ml of ethanol were added 1N sodium hydroxide (5 ml), glycyrrhizin (1 g), and ground roe of brine shrimp (0.1 g), and ethanol was added to make the total amount 100 ml. The solvent was evaporated under reduced pressure, and the resultant mixture was dried and pulverized into powder to give an agent for strengthening liver of cultured fish.

### Example 8

### Agent for treatment and prevention of vibriosis in sea water fish

Gellan gum (0.2 g) and food dye blue 2 (0.005 g) were mixed, and purified water (80 ml) was added thereto, followed by dissolution by heating. Then, sulfamonomethoxine (0.5 g) was dissolved therein, and fish feed flavor (0.1 ml) was added. The total amount was adjusted to 100 ml to give an agent for the treatment and prevention of vibriosis in sea water fish.

### Example 9

### Agent for treatment and prevention of placoid scale of cultured fish

Polyvinyl acetal diethylaminoacetate (5 g) was dissolved in 1N hydrochloric acid (0.6 ml), and purified water (about 80 ml) was added thereto. Then, sulfamonomethoxine (0.5 g) and food dye red 102 (0.005 g) were dissolved therein. The pH of the solution was adjusted to 5 with 1N sodium hydroxide. Ground roe of cod fish (0.1 g) was added thereto, and the mixture was lyophilized and pulverized into powder to give an agent for the treatment and prevention of placoid scale of cultured fish.

### Example 10

### Composition containing antibiotics for cultured fish

Cellulose acetate phthalate (4 g) was dissolved in 100 ml of ethanol, and added with 1N sodium hydroxide (0.2 ml), oxytetracycline hydrochloride (0.25 g), and ground roe of brine shrimp (0.1 g), followed by evaporation of ethanol under reduced pressure. After drying, the mixture was pulverized into powder to give a composition containing antibiotics for cultured fish.

The composition to be fed to fish and shellfish of the present invention comprising feed, nutrition furnisher, and agent for treatment and prevention of diseases can be effectively fed to fish and shellfish while suppressing water pollution, by the action of a polymer compound comprised in the composition, which is formed into particles upon liquid-gel phase transition caused by the change of ionic strength or change of pH of the water for raising fish and shellfish; in which particles, feed, nutrition furnisher, and agent for treatment and prevention of diseases are entrapped, thereby effectively inhibiting those components from dissolving in and eluting out into water.

## Claims

1. A composition to be fed to fish and shellfish, comprising a polymer compound capable of liquid-gel phase transition in the water for raising fish and shellfish.

2. The composition as claimed in Claim 1, wherein the polymer compound is capable of liquid-gel phase transition by change of ionic strength or change of pH.

3. The composition as claimed in Claim 1, wherein the polymer compound becomes gel at an ionic strength of not less than 0.2.

4. The composition as claimed in Claim 3, wherein the polymer compound is at least one polysaccharide selected from the group consisting of arginic acid, a salt thereof, κ-carageenan, and gellan gum.

5. The composition as claimed in Claim 1, wherein the polymer compound becomes gel at a pH of from 5 to 9.

6. The composition as claimed in Claim 5, wherein the polymer compound is at least one compound selected from the group consisting of acrylic resins, polyvinyl esters, cellulose esters, and hydroxyalkylcelluloses.

7. The composition as claimed in any one of claims 1-6, wherein the composition to be fed to fish and shellfish is a feed composition for fish and shellfish.

8. The composition as claimed in any one of claims 1-6, wherein the composition to be fed to fish and shellfish is a nutrition furnisher for fish and shellfish.

9. The composition as claimed in any one of claims 1-6, wherein the composition to be fed to fish and shellfish is an agent for the treatment and prevention of diseases in fish and shellfish.

10. A method for suppressing pollution of the water for raising fish and shellfish, comprising addition of a polymer compound capable of liquid-gel phase transition by change of ionic strength or change of pH, and administration substance for fish and shellfish, to the water for raising fish and shellfish.
